# EUROPÄISCHE PATENTSCHRIFT

(11) **EP 0 616 991 B1**
(45) Veröffentlichungstag und Bekanntmachung des Hinweises auf die Patenterteilung: **23.12.1998**
(21) Anmeldenummer: 94103274.0
(22) Anmeldetag: 04.03.1994
(51) Int. Cl.: C07B 63/04, C07C 201/16, C07C 17/38, B01J 43/00

(54) **Verfahren zur Reinigung von durch gelöste Metallverbindungen verunreinigten organischen Verbindungen**
Process for the preparation of organic compounds polluted by dissolved metallic compounds
Procédé pour la purification de composés organiques pollués en composés métalliques sous forme dissoute

(30) Priorität: 18.03.1993 DE 4308569
(43) Veröffentlichungstag der Anmeldung: 28.09.1994
(73) Patentinhaber: Clariant GmbH, 65929 Frankfurt am Main (DE)
(72) Erfinder: Leupold, Ernst Ingo, Dr., D-61267 Neu Anspach (DE); Dettmeier, Dr., D-65779 Kelkheim (Taunus) (DE); Gimpel, Gustav, D-65931 Frankfurt am Main (DE); Reimann, Walter, Dr., D-65719 Hofheim/Ts (DE)

(56) Entgegenhaltungen:
- FR-A- 1 530 321
- US-A- 3 475 495
- JOURNAL OF APPLIED CHEMISTRY OF USSR, Band 51, Nr. 1, 1978, New York, NY (US); V.S. SOLDATOV et al., Seiten 73-77

## Beschreibung

Die vorliegende Erfindung betrifft ein Verfahren zur Reinigung von organischen Verbindungen, die durch gelöste Metallverbindungen verunreinigt sind. Die verunreinigenden Metallverbindungen gelangen einerseits bedingt durch die Art der Herstellung der organischen Verbindungen und/oder andererseits durch eine korrodierende Wirkung, welche die organischen Verbindungen auf die Wände von Rohrleitungen und Reaktorgefäßen ausüben, in die organische Verbindung. In einer Reihe von Fällen sind die Verunreinigungen auf die Verwendung von Metallen oder Metallverbindungen enthaltenden Katalysatoren zurückzuführen. Ohne den Anspruch auf Vollständigkeit zu erheben, seien als Beispiele hierfür die Hydroformylierung von Olefinen in Gegenwart von entsprechenden Katalysatoren, die katalytische Hydrierung von Aldehyden, Olefinen und Azomethinen in Gegenwart fest angeordneter Hydrierkatalysatoren sowie Synthesen, die mit Hilfe von Lewis-Säuren durchgeführt werden, genannt.

So enthalten halogenierte organische Verbindungen, insbesondere halogenierte bzw. chlorierte aromatische Verbindungen - verursacht durch die Art ihrer Herstellung - häufig geringe Mengen von Metallverbindungen, beispielsweise FeCl₃, die bei der Halogenierung bzw. Chlorierung der aromatischen Ausgangsverbindungen als Katalysator zugesetzt werden.

Da organische Verbindungen in einer Reihe von Fällen auch auf die Wandmaterialien der verwendeten Rohrleitungen und Reaktorgefäße einen korrodierenden Einfluß ausüben, finden sich in den organischen Verbindungen auch Metallverbindungen, die aus dem Wandmaterial entstammen. Bei der Weiterverarbeitung von durch gelöste Metallverbindungen verunreinigten organischen Verbindungen erweisen sich die Verunreinigungen, die beispielsweise zu einer Verfärbung der Endprodukte führen oder in nachfolgenden Reaktionsschritten, beispielsweise in der katalytischen Hydrierung von Chlornitroaromaten, Störungen verursachen können, als unerwünscht. Dies betrifft beispielsweise die Verwendung von organischen Verbindungen zur Herstellung von Feinchemikalien, Farben, Pflanzenschutzmitteln, ebenso wie ihr Einsatz zur Herstellung von Polymeren oder Pharmazeutika, an deren Reinheit besonders hohe Anforderungen gestellt werden.
Aus diesem Grunde müssen die verunreinigenden Metallverbindungen möglichst weitgehend entfernt werden.

Bislang sind nur wenige, aufwendige Methoden zur Entfernung solcher Verunreinigungen aus nicht-wäßrigen System bekannt. So beschreibt die US-PS 3 351 669 die Reinigung von Halogenphenolen, die einen Eisenhalogenidgehalt von 0,001 bis 2 Gew-% aufweisen, durch Behandlung mittels eines Anionenaustauscherharzes, das quarternäre Ammoniumgruppen enthält. Bei diesem Verfahren ist sicherzustellen, daß Eisen nicht als FeCl₃, sondern als FeCl₄⁻ vorliegt. Dies wird durch einen Zusatz von HCl gewährleistet. Weiterhin ist ein gewisser Anteil von Wasser erforderlich, um die Bildung der Metallhalogenid-Komplexe und somit deren Abtrennung zu ermöglichen.

Der Zusatz von HCl und Wasser hat jedoch erhebliche verfahrenstechnische Nachteile zur Folge. Zum einen begünstigen sowohl HCl als auch Wasser (in Verbindung mit Phenolen) Korrosionsvorgänge, zum anderen ergibt sich die Notwendigkeit, diese Zusätze vor einer Weiterverarbeitung möglichst weitgehend wieder entfernen zu müssen.

Es bestand daher die Aufgabe, ein Verfahren zur Reinigung von organischen Verbindungen zu entwickeln, daß erstens diese Nachteile vermeidet, zweitens sich in technisch einfacher Weise ausüben läßt und drittens mit hoher Selektivität selbst sehr kleine Mengen von Metallverbindungen entfernt.

Diese Aufgabe wird gelöst durch ein Verfahren zur Reinigung von durch gelöste Metallverbindungen verunreinigten organischen Verbindungen. Es ist dadurch gekennzeichnet, daß man die verunreinigte organische Verbindung mit einem lonenaustauscherharz auf Basis von Polyacrylaten, Polyacrylsäuren, Polyacrylamiden, Phenol-Formaldehyd-Harzen, Polyalkylaminharzen oder auf Basis eines durch Divinylbenzol vernetzten Polystyrols, dessen funktionelle Gruppe sowohl eine sekundäre Amingruppe als auch einen Phosphonsäuresalzrest und/oder Phosphonsäurerest enthält, in Kontakt bringt.

Das erfindungsgemäße Verfahren läßt sich technisch recht einfach realisieren, indem man beispielsweise das lonenaustauscherharz in Form eines Festbettes anordnet und die verunreinigte organische Verbindung darüberleitet. Diese Arbeitsweise eignet sich besonders für ein kontinuierliches Verfahren.

Es ist allerdings auch möglich, das lonenaustauscherharz der verunreinigten organischen Verbindung unter Vermischen beizufügen und anschließend lonenaustauscherharz und organische Verbindung durch Sedimentieren voneinander zu trennen. Dieses Vorgehen empfiehlt sich für eine diskontinuierliche Arbeitsweise.

Zur Durchführung des Verfahrens kann sowohl auf die Zugabe von HCI als auch auf einen Zusatz von Wasser verzichtet werden. Somit entfällt auch eine nachträgliche Abtrennung dieser zugesetzten Stoffe. Die verunreinigten organischen Verbindungen enthalten Wasser entweder entsprechend seiner Löslichkeit in sehr geringen Mengen oder gar nicht. Von besonderem Vorteil ist, daß sich das Verfahren in Abwesenheit von Wasser ausführen läßt.

Ein weiterer Vorteil des Verfahrens besteht darin, daß selbst geringe Mengen Metallverbindung, beispielsweise ≤ 100, insbesondere ≤ 50 ppm nicht nur mit hoher Selektivität, sondern auch in sehr großem Umfange aus der organischen Verbindung abgetrennt werden. Selbst Mengen von ≤ 10 ppm Metallverbindung lassen sich mittels des erfindungsgemäßen Verfahrens noch in gewünschtem Umfange (≥ 90 %) entfernen.

Die organische Verbindung enthält als Verunreinigung ein Eisen-, Nickel-, Chrom-, Vanadium-, Kobalt-, Kupfer-, Zink-, und/oder Bleisalz, insbesondere ein Eisen-, Nickel-, Chrom- und/oder Kobaltsalz, bevorzugt ein Eisen-, Nickel- und/oder Chromsalz in gelöster Form.

Das Verfahren eignet sich zur Reinigung beliebiger organischer Verbindungen, die unter den Verfahrensbedingungen flüssig vorliegen. Beispiele für geeignete organische Verbindungen sind aliphatische Kohlenwasserstoffe, Olefine, Aldehyde, Amine, Alkohole, chlorierte aliphatische und aromatische Kohlenwasserstoffe oder deren Gemische. In das Verfahren lassen sich mit gutem Erfolg halogenierte organische Verbindungen einsetzen. Das Verfahren eignet sich in besonderem Maße zur Reinigung von halogenierten aromatischen Verbindungen, insbesondere chlorierten oder bromierten aromatischen Verbindungen. Geeignete halogenierte aromatische Verbindungen sind beispielsweise gegebenenfalls substituierte, chlorierte Nitrobenzole, insbesondere ortho- meta- und para-Chlornitrobenzol, bevorzugt meta-Chlornitrobenzol.

Das verwendete Ionenaustauscherharz soll sich unter den vorliegenden Verfahrensbedingungen inert verhalten, d.h. es darf nicht mit dem zu reinigendem Substrat reagieren. Für viele Fälle eignet sich ein Ionenaustauscherharz auf Basis eines durch Divinylbenzol vernetzten Polystyrols. Es lassen sich jedoch auch andere übliche Harze, beispielsweise Polyacrylate, Polyacrylsäure, Polyacrylamide, Phenol-Formaldehydharze oder Polyalkylaminharze einsetzen.

Durch Durchführung des Verfahrens empfiehlt es sich, ein Ionenaustauscherharz zu verwenden, dessen funktionelle Gruppe der Formel -(CH₂)ₘNH(CH₂)ₙPO₃X₂ entspricht, worin m und n unabhängig voneinander jeweils für eine ganze Zahl von 1 bis 8, insbesondere 1 bis 4 und X für ein Wasserstoffatom oder ein Metallatom, insbesondere ein Alkalimetallatom, steht.

Besonders gut geeignet ist ein Ionenaustauscherharz, dessen funktionelle Gruppe der Formel -CH₂NHCH₂PO₃Na₂ entspricht.

Das erfindungsgemäße Verfahren läßt sich in einem relativ breiten Temperaturbereich, beispielsweise von 0 bis 100, insbesondere 20 bis 80°C durchführen. Im allgemeinen wählt man die Verfahrensbedingungen (Druck und Temperatur) derart, daß die organische Verbindung im flüssigen oder gegebenenfalls im gelösten Zustande vorliegt.

Das Verfahren erfordert keinen besonderen technischen Aufwand. An die Apparaturen, die sich für eine Durchführung der Reinigung anbieten, werden keine besonderen Anforderungen gestellt. Das Verfahren kann in allen Apparaturen, die sich für die Durchführung von Reaktionen im flüssigen Zustand eignen, durchgeführt werden, beispielsweise - wie bereits zuvor erwähnt - kontinuierlich in einem mit dem Ionenaustauscherharz gefüllten, gegebenenfalls beheizbaren Strömungsrohr oder diskontinuierlich in einem Rührbehälter, in dem das Ionenaustauscherharz und die zu reinigende organische Verbindung zu einer Suspension vermischt werden.

Die nachfolgenden Beispiele belegen die Erfindung, ohne sie zu beschränken.

### Beispiel 1

Es wird ein handelsübliches Ionenaustauscherharz eingesetzt,dessen Matrix aus Polystyrol, vernetzt mit Divinylbenzol, besteht und eine makroporöse Struktur besitzt. Als funktionelle Gruppe enthält das Harz das Strukturelement -CH₂NHCH₂PO₃Na₂.

30 ml Ionenaustauscherharz werden nach Auswaschen mit Methanol und Vakuumtrocknung in ein senkrecht angeordnetes beheizbares Rohr (Innendurchmesser: 1,2 cm; Länge: 35 cm), das am unteren Ende mit einem Hahn versehen ist, gefüllt. Zunächst wird das Rohr mit meta-Chlornitrobenzol gefüllt, das 10 ppm Eisen in Form von FeCl₃ enthält. Auf den oberen Teil des Rohres wird ein mit demselben Produkt gefüllter Tropftrichter gesetzt. Rohr und Tropftrichter werden durch Mantelheizung auf 60°C gehalten. Durch Öffnen des Hahns wird das Eluat in einer Geschwindigkeit von 60 ml/h (LHSV: 2 h⁻¹) abgenommen:

| Fe-Konzentration im Eluat | |
|---|---|
| Laufzeit (Stunden) | ppm Fe |
| 10 | < 1 |
| 25 | < 1 |
| 50 | < 1 |
| 75 | < 1 |
| 100 | < 1 |

Auch nach weit über 100 Stunden erhält man ein nahezu eisenfreies Eluat.

### Beispiel 2

In einem beheizbaren Rührgefäß wird 1 kg m-Chlornitrobenzol (Fe-Gehalt: 6 ppm; Fe als FeCl₃) bei 50°C mit 1,5 g des im Beispiel 1 beschriebenen Harzes intensiv gerührt. Es werden stündlich Proben entnommen und analysiert:

| Fe-Konzentration im Produkt: | |
|---|---|
| Laufzeit (Stunden) | ppm Fe |
| 1 | 3,3 |
| 2 | < 1 |

### Vergleichsbeispiel

Es wird, wie in Beispiel 2 beschrieben (Fe-Gehalt: 6 ppm; Fe als FeCl₃), verfahren, jedoch werden verschiedene Ionenaustauscherharze eingesetzt. Die Ergebnisse sind der nachfolgenden Zusammenstellung zu entnehmen:

| Nr. | Funktionelle Gruppe | ppm Fe nach 5 Stunden Reaktionszeit |
|---|---|---|
| A | wie Beispiele 1 und 2 | < 1 |
| B | -NHR | 3,5 |
| C | -NR₃ + Cl⁻ (nach US 3,351,669) | 2,8 |
| D | -SO₃H | 5,3 |
| E | -CH₂-NH₂ | 4,9 |

Wie aus den Vergleichsbeispielen B bis E hervorgeht, verläuft die Abtrennung des FeCl₃ nur sehr unvollständig.

## Patentansprüche

1. Verfahren zur Reinigung von durch gelöste Metallverbindungen verunreinigten organischen Verbindungen, dadurch gekennzeichnet, daß man die verunreinigte organische Verbindung mit einem lonenaustauscherharz auf Basis von Polyacrylaten, Polyacrylsäuren, Polyacrylamiden, Phenol-Formaldehyd-Harzen, Polyalkylaminharzen oder auf Basis eines durch Divinylbenzol vernetzten Polystyrols,dessen funktionelle Gruppe sowohl eine sekundäre Amingruppe als auch einen Phosphonsäuresalzrest und/oder Phosphonsäurerest enthält, in Kontakt bringt.

2. Verfahren nach Anspruch 1, dadurch gekennzeichnet, daß die verunreinigte organische Verbindung ein Eisen-, Nickel-, Chrom-, Cobalt-, Kupfer- und/oder Bleisalz gelöst, enthält.

3. Verfahren nach Anspruch 1 oder 2, dadurch gekennzeichnet, daß man als verunreinigte organische Verbindung eine halogenierte organische Verbindung einsetzt.

4. Verfahren nach einem oder mehreren der Ansprüche 1 bis 3, dadurch gekennzeichnet, daß man als verunreinigte organische Verbindung eine halogenierte aromatische Verbindung einsetzt.

5. Verfahren nach einem oder mehreren der Ansprüche 1 bis 3, dadurch gekennzeichnet, daß man als verunreinigte organische Verbindung ein gegebenenfalls substituiertes, chloriertes Nitrobenzol, insbesondere m-Chlornitrobenzol einsetzt.

6. Verfahren nach einem oder mehreren der Ansprüche 1 bis 5, dadurch gekennzeichnet, daß man ein lonenaustauscherharz auf Basis eines durch Divinylbenzol vernetzten Polystyrols einsetzt.

7. Verfahren nach einem oder mehreren der Ansprüche 1 bis 6, dadurch gekennzeichnet, daß die funktionelle Gruppe des lonenaustauscherharzes der Formel -(CH₂)ₘNH(CH₂)ₙPO₃X₂ entspricht, worin m und n unabhängig voneinander jeweils für eine ganze Zahl von 1 bis 8, insbesondere 1 bis 4, und X für ein Wasserstoffatom oder ein Metallatom, insbesondere ein Alkalimetallatom steht.

8. Verfahren nach einem oder mehreren der Ansprüche 1 bis 7, dadurch gekennzeichnet, daß die funktionelle Gruppe der Formel -CH₂NHCH₂PO₃Na₂ entspricht.

9. Verfahren nach einem oder mehreren der Ansprüche 1 bis 8, dadurch gekenneichnet, daß man die organische Verbindung mit dem Ionenaustauscherharz bei 0 bis 100, insbesondere bei 20 bis 80°C in Kontakt bringt.

## Claims

1. A process for the purification of organic compounds contaminated by dissolved metal compounds, which comprises bringing the contaminated organic compound into contact with an ion exchanger resin based on polyacrylates, polyacrylic acids, polyacrylamides, phenol-formaldehyde resins, polyalkylamine resins or based on a divinylbenzene-crosslinked polystyrene, the functional group of which ion exchanger resin contains both a secondary amine group and a phosphonate radical and/or phosphonic acid radical.

2. The process as claimed in claim 1, wherein the contaminated organic compound contains a dissolved iron, nickel, chromium, cobalt, copper and/or lead salt.

3. The process as claimed in claim 1 or 2, wherein the contaminated organic compound used is a halogenated organic compound.

4. The process as claimed in one or more of claims 1 to 3, wherein the contaminated organic compound used is a halogenated aromatic compound.

5. The process as claimed in one or more of claims 1 to 3, wherein the contaminated organic compound used is an unsubstituted or substituted chlorinated nitrobenzene, in particular m-chloronitrobenzene.

6. The process as claimed in one or more of claims 1 to 5, wherein an ion exchanger resin based on a divinylbenzene-crosslinked polystyrene is used.

7. The process as claimed in one or more of claims 1 to 6, wherein the functional group of the ion exchanger resin corresponds to the formula -(CH₂)ₘNH(CH₂)ₙPO₃X₂, in which m and n, independently of each other, are respectively an integer from 1 to 8, in particular 1 to 4, and X is a hydrogen atom or a metal atom, in particular an alkali metal atom.

8. The process as claimed in one or more of claims 1 to 7, wherein the functional group corresponds to the formula -CH₂NHCH₂PO₃Na₂.

9. The process as claimed in one or more of claims 1 to 8, wherein the organic compound is brought into contact with the ion exchanger resin at 0 to 100, in particular at 20 to 80°C.

## Revendications

1. Procédé de purification de composés organiques souillés par des composés métalliques dissous, caractérisé en ce que l'on met en contact les composés organiques souillés avec une résine échangeuse d'ions à base de polyacrylates, d'acides polyacryliques, de polyacrylamides, de résines phénol-formaldéhyde, de résines de polyalkylamines ou à base d'un polystyrène réticulé par le divinylbenzène, dont le groupe fonctionnel contient aussi bien un groupe secondaire, qu'un reste d'un sel d'acide phosphorique et/ou un reste d'acide phosphonique.

2. Procédé selon la revendication 1, caractérisé en ce que le composé organique souillé contient un sel de fer, de nickel, de chrome, de cobalt, de cuivre et/ou de plomb.

3. Procédé selon la revendication 1 ou 2, caractérisé en ce que l'on utilise comme composé organique souillé un composé organique halogéné.

4. Procédé selon une ou plusieurs des revendications 1 à 3, caractérisé en ce que l'on utilise comme composé organique souillé un composé aromatique halogéné.

5. Procédé selon une ou, plusieurs des revendications 1 à 3, caractérisé en ce que l'on utilise comme composé organique souillé un nitrobenzène chloré éventuellement substitué, en particulier le m-chloronitrobenzène.

6. Procédé selon une ou plusieurs des revendications 1 à 5, caractérisé en ce que l'on utilise une résine échangeuse d'ions à base d'un polystyrène réticulé par le divinylbenzène.

7. Procédé selon une ou plusieurs des revendications 1 à 6, caractérisé en ce que le groupe fonctionnel de la résine échangeuse d'ions correspond à la formule -(CH₂)ₘNH(CH₂)mPO₃X₂, dans laquelle m et n, indépendamment l'un de l'autre, représentent chacun un nombre entier de 1 à 8, en particulier de 1 à 4, et X représente un atome d'hydrogène ou un atome métallique, notamment un atome de métal alcalin.

8. Procédé selon une ou plusieurs des revendications 1 à 7, caractérisé en ce que le groupe fonctionnel répond à la formule -CH₂NHCH₂PO₃Na₂.

9. Procédé selon une ou plusieurs des revendications 1 à 8, caractérisé en ce que l'on met en contact le composé organique avec la résine échangeuse d'ions, à une température de 0 à 100 °C, notamme à 20 à 80 °C.
